# FASCICULE DE BREVET EUROPEEN

(11) **EP 3 320 462 B1**
(45) Date de publication et mention de la délivrance du brevet: **07.04.2021**
(21) Numéro de dépôt: 16739049.1
(22) Date de dépôt: 01.07.2016
(51) Int. Cl.: G16H 50/20

(54) **PROCEDE D'AIDE AU DIAGNOSTIC MEDICAL**
VERFAHREN ZUR UNTERSTÜTZUNG EINER MEDIZINISCHEN DIAGNOSE
MEDICAL DIAGNOSIS ASSISTANCE METHOD

(30) Priorité: 10.07.2015 FR 1556591
(43) Date de publication de la demande: 16.05.2018
(73) Titulaire: SORBONNE UNIVERSITE, 75006 Paris (FR); INSERM (Institut National de la Santé et de la Recherche Médicale), 75013 Paris (FR); UCL Business PLC, London W1T 4TP (GB); Assistance Publique - Hôpitaux de Paris, 75004 Paris (FR)
(72) Inventeur: DHOMBRES, Ferdinand, 75020 Paris (FR); JOUANNIC, Jean-Marie, 75014 Paris (FR); JAUNIAUX, Eric, London SE23 3BW (GB); MALENGREZ, Pascal, 75007 Paris (FR); MAURICE, Paul, 75015 Paris (FR)
(74) Mandataire: IPAZ
(86) Numéro de dépôt international: PCT/EP2016/065492
(87) Numéro de publication internationale: WO 2017/009070

(56) Documents cités:
- US-A1- 2007 237 377
- US-A1- 2014 012 790
- Thèse / Université ET AL: "ANNÉE 2013 École doctorale Vie - Agro - Santé (VAS) présentée par Mme. Valérie BERTAUD-GOUNOT PU-PH -Université Rennes 1 / co-directeur", , 20 décembre 2013 (2013-12-20), XP055252158, Extrait de l'Internet: URL:https://ecm.univ-rennes1.fr/nuxeo/site /esupversions/2c20b520-4c5b-4b4c-a38b-ea64 d1314bef [extrait le 2016-02-22]

## Description

### Domaine technique

L'invention se situe dans le domaine de l'assistance à l'établissement d'un diagnostic médical. Elle s'applique notamment, mais pas exclusivement, à l'assistance au diagnostic des grossesses ectopiques au moyen d'un examen échographique. Plus précisément, l'invention concerne un procédé d'assistance à l'établissement d'un diagnostic d'un patient, à partir d'un signe identifié, en s'appuyant sur une base de connaissances informatique comportant une ontologie médicale.

### État de la technique antérieure

La technique échographique est une technique d'acquisition d'images relativement peu onéreuse, et considérée comme indolore et non invasive pour le patient et le fœtus. Son utilisation est aujourd'hui largement répandue dans le monde, aussi bien dans le domaine obstétrique que le domaine gynécologique.

Dans le domaine obstétrique, le dépistage prénatal des anomalies fœtales par échographie a connu un essor considérable dans les trente dernières années. Dans la majorité des pays développés, ce dépistage repose sur la réalisation d'un ou deux examens de routine réalisés vers cinq et sept mois d'aménorrhée par un opérateur, par exemple un médecin, une sage-femme, un radiologue ou un "sonographer", selon la terminologie anglo-saxonne. Un référentiel de coupes échographiques standardisées a progressivement été proposé, de manière à balayer les organes fœtaux et y déceler les principaux signes susceptibles de les affecter. Ces signes concernent par exemple une anomalie d'un organe du fœtus, telle qu'une malformation ou un retard de croissance, ou une anomalie de la quantité de liquide amniotique. Un excès de liquide amniotique (hydramnios) peut être le premier signe d'alarme d'une affection fœtale. Dans la situation où l'une des coupes échographiques ne correspond pas à l'image habituelle, autrement dit lorsque la présence d'un symptôme est suspectée, un contrôle immédiat ou différé est généralement réalisé par un opérateur expert. De même, l'analyse d'un expert peut être recommandée en cas d'antécédent familial, par exemple en cas d'antécédent de malformation, de couple présentant un risque élevé d'anomalie génétique, ou de pathologie maternelle antérieure à la grossesse exposant au risque d'embryo-fœtopathie. De façon générale, un examen détaillé, réalisé par un expert, s'avère recommandé dans environ 15 à 20% des grossesses. Le rôle de l'expert est alors d'infirmer ou de confirmer l'existence d'une ou plusieurs anomalies, puis de rassembler des éléments concourant à l'établissement d'un diagnostic et d'un pronostic.

Selon les pays et les établissements de soin, l'opérateur chargé de réaliser le diagnostic prénatal par échographie est plus ou moins expérimenté. La plupart du temps, et en particulier dans les structures d'urgences gynécologiques, ce sont de jeunes médecins, peu expérimentés en échographie, qui interviennent en première ligne. Le risque est que les premiers signes ne soient pas identifiés, et que la patiente ne soit pas dirigée en temps utile vers un expert.

Par ailleurs, l'état actuel des connaissances en matière de pathologie obstétrique entraîne une grande complexité dans l'établissement d'un diagnostic. Le nombre de maladie rares (environ 6000), le nombre d'anomalies de développement (environ 1000), et le nombre de signes (environ 8000 en postnatal), de plus en plus accessibles en prénatal avec l'amélioration des techniques d'imagerie, montrent que même un praticien expert ne peut maîtriser l'ensemble des connaissances du domaine.

Afin de gérer efficacement toutes ces connaissances, des systèmes experts ont été développés. Ces systèmes experts comportent une base de faits, en l'occurrence des signes et des états pathologiques, une base de règles, et un moteur d'inférence. En l'occurrence, les faits correspondent à des signes et à des états pathologiques, et les règles correspondent à des liens de corrélation entre les signes et les états pathologiques. Le moteur d'inférence s'apparente à une intelligence artificielle. Il utilise les différents faits et les règles entre ces faits pour générer de nouveaux faits, jusqu'à parvenir à une réponse les satisfaisant au mieux. Un inconvénient des systèmes experts est qu'un résultat, c'est-à-dire un diagnostic, ne peut être fourni que si l'ensemble des informations nécessaires sont disponibles. Or ces informations ne sont pas toujours identifiables par un praticien ayant peu d'expérience. En pratique, les systèmes experts peuvent donc faciliter l'établissement d'un diagnostic par un médecin expert, mais ne peuvent généralement pas assister un opérateur 3 ayant peu d'expérience dans la conduite de son examen, en vue de l'établissement d'un diagnostic, ultérieurement, par un médecin expert.

Le document US 2014/012790 A1 décrit une méthode pour aider un diagnostic clinique, permettant, à partir de symptômes initiaux, d'en déduire des maladies possibles puis des nouveaux symptômes associés. Une thèse, présentée par M. DONFACK GUEFACK, décrit des méthodes de modélisation des signes dans les ontologies biomédicales pour l'aide au diagnostic. Enfin, le document US 2007/237377 A1 décrit une méthode de création d'un support pour un compte rendu médical.

Dans le domaine gynécologique, l'échographie est devenue pour de nombreux gynécologues le prolongement naturel de l'examen clinique gynécologique. Elle permet notamment de diagnostiquer les pathologies gynécologiques les plus fréquentes, telles que l'endométriose, les kystes ovariens (bénins ou malins), les fibromes utérins, l'adénomyose utérine ou l'exploration de méno-métrorragies. Lorsque l'échographie pelvienne est réalisée par un opérateur expérimenté, elle conduit à une forte pertinence diagnostique.

Cependant, comme pour le dépistage prénatal des anomalies fœtales, la qualité diagnostique est en pratique médiocre en raison, d'une part, d'un défaut de formation des opérateurs et, d'autre part, de l'absence d'un protocole d'examen défini, contrairement à ce qui peut être observé dans le domaine obstétrique.

### Exposé de l'invention

L'invention est définie par les revendications jointes.

Un but de l'invention est notamment de remédier à tout ou partie des inconvénients précités.

En particulier, un but de l'invention est de permettre aux opérateurs réalisant un examen médical d'être assistés afin de récolter l'ensemble des informations nécessaires pour la pose d'un diagnostic fiable. Cela implique de collecter des informations non seulement pour corroborer le diagnostic établi, mais aussi pour écarter d'autres diagnostics envisageables.

L'invention vise notamment à permettre aux opérateurs ayant peu d'expérience et de connaissances dans le domaine considéré, de collecter l'ensemble des informations nécessaires à un expert pour lui permettre de poser un diagnostic fiable.

L'invention repose sur l'utilisation d'une base de connaissances informatique comportant une ontologie médicale. De manière générale, une ontologie médicale constitue une représentation logique des connaissances dans le domaine médical, ou dans un domaine médical particulier. Différents concepts, tels que des signes, des organes, des états pathologiques (maladies ou syndromes), sont reliés les uns aux autres ou entre eux au moyen de relations sémantiques. L'ontologie est généralement modélisée dans un langage standard du Web, interprétable par ordinateur, ce qui permet la réalisation de procédures automatiques. Les ontologies ont pour principal avantage de pouvoir relier des données hétérogènes, de permettre le traitement d'une quantité importante de données, et de pouvoir être enrichies de nouvelles données sans remettre en question la structure des données.

Plus précisément, l'invention a pour objet un procédé d'assistance à l'établissement d'un diagnostic d'un patient mis en œuvre par un dispositif, à partir d'au moins un signe identifié et saisi par un opérateur dans ce dispositif et en s'appuyant sur une base de connaissances informatique comportant une ontologie médicale, l'ontologie médicale comprenant :
▪ une liste de signes, formant une classe "signe",
▪ une liste d'états pathologiques, formant une classe "état pathologique", et
▪ un premier ensemble de relations logiques entre les signes et les états pathologiques, chaque relation logique établissant un lien de corrélation entre un signe et un état pathologique,
le procédé comprenant :
▪ une étape de recherche d'états pathologiques potentiels, dans laquelle l'ensemble des états pathologiques liés par un lien de corrélation avec au moins l'un des signes identifiés sont recherchés dans la classe "état pathologique" au moyen du premier ensemble de relations logiques, lesdits états pathologiques formant les états pathologiques potentiels, et
▪ une étape d'identification de signes potentiels, dans laquelle, pour chaque état pathologique potentiel, l'ensemble des signes liés par un lien de
▪ corrélation avec ledit état pathologique potentiel sont identifiés dans la classe "signe" au moyen du premier ensemble de relations logiques, lesdits signes formant les signes potentiels.

Dans la présente demande de brevet, le terme "signe" désigne un phénomène lié à l'état d'un patient. Il recouvre notamment les concepts de "signe fonctionnel", de "signe clinique", et de "signe paraclinique". Un signe fonctionnel, également appelé "symptôme", désigne un phénomène tel que ressenti et exprimé subjectivement, qualitativement ou quantitativement par le patient. Il s'agit par exemple d'une fatigue, d'une douleur, ou d'une toux. Un signe clinique est un signe observé lors de l'examen clinique d'un patient. Il est constaté objectivement, que le patient le perçoive ou non. Il s'agit par exemple d'un souffle au cœur, d'un réflexe anormal à un examen neurologique, d'un phénomène de crépitation observé dans le champ pulmonaire, ou d'une démarche ébrieuse d'un patient marchant les yeux fermés. Un signe paraclinique est un signe observé à l'aide d'un instrument. Il s'agit par exemple d'un signe biologique, d'un signe histologique (anatomie pathologique d'un cancer par exemple), ou d'un signe d'imagerie, c'est-à-dire un signe observé lors de l'interprétation d'une image médicale. Une image médicale peut être obtenue par différentes techniques d'acquisition d'images, telles que la radiographie, l'échographie, le scanner, ou l'imagerie par résonnance magnétique.

Dans la présente demande de brevet, le terme "état pathologique" désigne un état d'altération des fonctions, de la morphologie ou de la santé d'un organe ou organisme dont on connait ou non la cause, et qui se caractérise par la présence ou l'absence d'un ou plusieurs signes. À noter que l'état pathologique est désigné par le terme "maladie" lorsque l'on connaît la cause, et par le terme "syndrome" sinon.

Le procédé selon l'invention permet ainsi, à partir d'un ou plusieurs signes identifiés, de remonter à l'ensemble des états pathologiques affectant potentiellement le patient, et de lister l'ensemble des signes corrélés à chacun de ces états pathologiques possibles, afin de proposer au praticien une liste de signes pouvant être vérifiés et lui permettre, après analyse, de sélectionner un ou plusieurs états pathologiques affectant potentiellement le patient.

Selon une première variante de réalisation, dans le cas où plusieurs signes identifiés sont utilisés dans le procédé, l'étape de recherche d'états pathologiques potentiels est réalisée en considérant individuellement chaque signe identifié. L'étape de recherche d'états pathologiques potentiels consiste alors à rechercher, pour chaque signe identifié, l'ensemble des états pathologiques liés par un lien de corrélation avec le signe identifié considéré, les différents ensembles d'états pathologiques ainsi trouvés formant les états pathologiques potentiels. L'étape d'identification de signes potentiels peut être réalisée plusieurs fois, après chaque recherche d'états pathologiques liés à l'un des signes identifiés, ou une seule fois, lorsque tous les états pathologiques potentiels ont été trouvés.

Selon une deuxième variante de réalisation, l'étape de recherche d'états pathologiques potentiels est réalisée en considérant collectivement l'ensemble des signes identifiés. L'étape de recherche d'états pathologiques potentiels consiste alors à rechercher l'ensemble des états pathologiques liés par un lien de corrélation avec chacun des signes identifiés.

Selon une forme particulière de réalisation, le premier ensemble de relations logiques comprend des relations logiques selon chacune desquelles un signe est révélateur d'au moins un état pathologique. Autrement dit, une relation logique peut prendre la forme suivante : "Le signe A est révélateur de l'état pathologique B". Une relation logique peut relier un unique signe à un unique état pathologique. Lorsqu'un signe donné est révélateur de plusieurs états pathologiques, une multiplicité de relations logiques relient ce signe aux états pathologiques. L'ontologie permet de représenter une relation logique définissant plusieurs liens entre un signe donné et des états pathologiques. La relation logique prend alors par exemple la forme suivante : Le signe C est révélateur des états pathologiques D, E et F". D'autres types de relations logiques sont également possibles. Des signes ne sont par exemple jamais observés dans certains états pathologiques. La relation logique correspondante peut alors prendre la forme suivante : "Le signe G est révélateur de l'absence de l'état pathologique H". Il est possible d'attribuer à chaque relation logique un coefficient représentatif de la probabilité qu'un signe soit associé à un état pathologique. La relation logique prend alors la forme suivante : "Le signe J est présent dans 80% des cas de l'état pathologique K".

Les états pathologiques peuvent présenter certaines corrélations entre eux, notamment des relations de parenté. Autrement dit, certains états pathologiques peuvent être constitutifs d'un ou plusieurs états pathologiques. On parle dans ce cas d'ordre entre les états pathologiques. Pour un état pathologique d'ordre m donné, où m est un entier naturel strictement positif, tous les états pathologiques auxquels il appartient forment les états pathologiques parents, d'ordre p, avec p un entier naturel strictement supérieur à m. À titre d'exemple, l'état pathologique "rhume" peut être considéré comme un état pathologique d'ordre un, constitutif d'un état pathologique d'ordre deux, "affection virale du nez". Cet état pathologique d'ordre deux est lui-même constitutif d'un état pathologique d'ordre trois, "affection virale". Cet état pathologique est constitutif d'un état pathologique d'ordre quatre, "maladie infectieuse".

Ainsi, selon une forme particulière de réalisation, l'ontologie comprend, en outre, un deuxième ensemble de relations logiques entre des états pathologiques de la classe " état pathologique", chaque relation logique définissant un lien de parenté entre un état pathologique donné et un état pathologique parent de cet état pathologique donné, l'étape de recherche d'états pathologiques potentiels comprenant :
▪ une recherche d'états pathologiques potentiels d'ordre un, dans laquelle l'ensemble des états pathologiques révélés par au moins l'un des signes identifiés sont recherchés, lesdits états pathologiques formant les états pathologiques potentiels d'ordre un, et
▪ une recherche d'états pathologiques potentiels d'ordre deux à N, où N est un entier naturel supérieur ou égal à deux, la recherche d'états pathologiques potentiels d'ordre n, où n est un entier naturel compris entre deux et N, comprenant, pour chaque état pathologique potentiel d'ordre n-1, une recherche de l'ensemble des états pathologiques parents de cet état pathologique potentiel d'ordre n-1 au moyen du deuxième ensemble de relations logiques, lesdits états pathologiques parents formant les états pathologiques potentiels d'ordre n,
l'étape d'identification de signes potentiels comprenant une identification de l'ensemble des signes révélateurs d'un état pathologique potentiel d'ordre un, et/ou d'un état pathologique potentiel d'ordre n.

La recherche d'états pathologiques d'ordre supérieur à l'ordre un est bornée par le nombre N. Ce nombre N doit être déterminé de manière à balayer un ensemble suffisant et raisonnable d'états pathologiques. Le nombre N peut ainsi être fixé en fonction de l'ontologie médicale considérée, et notamment en fonction du nombre d'états pathologiques dans cette ontologie, ou en fonction du nombre d'états pathologiques potentiels identifiés dans l'étape correspondante. Le nombre N est par exemple compris entre deux et quatre.

Avantageusement, l'étape d'identification de signes potentiels comprend un classement de ces signes potentiels, selon lequel l'ensemble des signes potentiels révélateurs d'un état pathologique potentiel d'ordre m, où m est un entier naturel compris entre un et N, forment les signes potentiels d'ordre m. Autrement dit, tous les signes potentiels identifiés directement depuis un état pathologique potentiel d'ordre n sont classés comme des signes potentiels d'ordre n. Dans la mesure où un même signe peut être révélateur de différents états pathologiques, certains signes identiques peuvent être classés avec des ordres différents. Il est alors possible de ne conserver que l'un de ces signes, de préférence celui associé à l'ordre le plus faible.

Afin d'aider le praticien à vérifier les différents signes potentiels, il est possible de lui indiquer les modalités techniques permettant d'observer ces signes potentiels. Tel est notamment le cas lorsque les signes peuvent être observés par imagerie médicale. L'ontologie médicale peut alors comprendre, en outre :
▪ une liste de modalités techniques d'acquisition d'images médicales, formant une classe "imagerie", chaque modalité technique d'acquisition d'une image médicale définissant un type de technologie d'acquisition d'images médicales, et un positionnement de l'image médicale sur le patient, et
▪ un troisième ensemble de relations logiques entre les signes et les modalités techniques d'acquisition d'images médicales, chaque relation logique définissant une modalité technique d'acquisition d'une image médicale permettant d'observer un signe,
le procédé comprenant, en outre :
▪ une étape d'identification de modalités techniques d'acquisition d'intérêt, dans laquelle, pour chaque signe potentiel, l'ensemble des modalités techniques d'acquisition d'images médicales permettant d'observer ledit signe potentiel sont identifiées dans la classe imagerie au moyen du troisième ensemble de relations logiques, lesdites modalités techniques d'acquisition d'images médicales formant les modalités techniques d'acquisition d'intérêt.

Le terme "modalité technique d'acquisition d'une image médicale", ou plus simplement "modalité d'acquisition", désigne un ensemble de particularités techniques permettant d'obtenir une certaine image médicale, apte à être comparée avec une image de référence ou des données de référence. En particulier, une modalité d'acquisition peut être définie par un type de technologie d'acquisition des images médicales, et un positionnement de l'image médicale relativement au patient. À titre d'exemples, une modalité d'acquisition peut être définie par la technique échographique, et une coupe sagittale médiane de l'encéphale du fœtus, ou une coupe du périmètre céphalique du fœtus, ou une coupe sagittale de l'utérus de la patiente.

L'étape d'identification de modalités techniques d'acquisition d'intérêt peut comprendre un classement de ces modalités techniques d'acquisition d'intérêt, selon lequel l'ensemble des modalités techniques d'acquisition d'images médicales d'intérêt permettant d'observer un signe potentiel d'ordre m forment les modalités techniques d'acquisition d'intérêt d'ordre m.

Le procédé d'assistance à l'établissement d'un diagnostic selon l'invention peut comporter une étape de construction d'un protocole d'examen. Le protocole d'examen comprend essentiellement une liste ordonnée des modalités techniques d'acquisition d'intérêt à proposer au praticien pour la recherche des signes potentiels. De préférence, les modalités techniques d'acquisition d'intérêt sont hiérarchisées selon leur ordre, les modalités techniques d'acquisition d'intérêt d'ordre m étant prioritaires par rapport aux modalités techniques d'acquisition d'intérêt d'ordre m+1.

Plusieurs modalités techniques d'acquisition d'intérêt peuvent avoir le même ordre. Dans ce cas, il est possible de privilégier les modalités techniques d'acquisition d'intérêt permettant d'observer le plus de signes potentiels. Ainsi, le procédé peut être agencé de sorte que les modalités techniques d'acquisition d'intérêt de même ordre sont hiérarchisées selon leur occurrence parmi l'ensemble des modalités techniques d'acquisition d'intérêt de cet ordre, ou parmi l'ensemble des modalités techniques d'acquisition d'intérêt des différents ordres.

Le procédé selon l'invention peut comprendre, en outre, une étape d'affichage des modalités techniques d'acquisition d'intérêt.

Le procédé peut également comporter :
▪ une étape de sélection de modalités techniques d'acquisition à mettre en œuvre, dans laquelle au moins l'une des modalités techniques d'acquisition d'intérêt est sélectionnée, l'ensemble des modalités techniques d'acquisition d'intérêt sélectionnées formant les modalités techniques d'acquisition à mettre en œuvre, et
▪ une étape d'acquisition d'une ou plusieurs images médicales, chaque image médicale étant acquise selon l'une des modalités techniques d'acquisition à mettre en œuvre.

L'étape d'acquisition d'une ou plusieurs images médicales peut être réalisée de manière plus ou moins automatique. À titre d'exemple, dans le cas où toutes les modalités techniques d'acquisition d'intérêt impliquent l'utilisation d'un dispositif d'imagerie par résonance magnétique, les différentes images peuvent être acquises sans nécessiter de déplacer le patient, et sans intervention du praticien.

Le procédé d'assistance à l'établissement d'un diagnostic d'un patient peut constituer un procédé itératif. En effet, une fois qu'un ensemble de signes potentiels ont été identifiés, il est possible de vérifier la présence ou l'absence de ces signes, et de les utiliser comme constituant les signes identifiés pour une nouvelle itération du procédé.

Ainsi, selon une forme particulière de réalisation, le procédé d'assistance à l'établissement d'un diagnostic d'un patient comporte une répétition des étapes de recherche d'états pathologiques potentiels, d'identification de signes potentiels et, le cas échéant, une répétition des étapes d'identification de modalités techniques d'acquisition d'intérêt, de sélection de modalités techniques d'acquisition à mettre en œuvre, et d'acquisition d'une ou plusieurs images médicales. Le procédé comporte par exemple, à l'issue de chaque itération de l'étape d'acquisition d'une ou plusieurs images médicales, une étape de vérification de l'état clinique des signes potentiels, ces signes potentiels constituant les signes identifiés pour une nouvelle itération du procédé, et une nouvelle itération des différentes étapes du procédé.

Toujours dans le but d'assister le praticien dans l'établissement des informations permettant d'établir un diagnostic, le procédé selon l'invention peut fournir au praticien des images de référence, aussi bien pour lui permettre de vérifier le caractère normal ou anormal d'un phénomène observé sur le patient, ou de l'aider à acquérir des images exploitables pour le diagnostic.

La base de connaissances informatique peut ainsi comporter, en outre, une bibliothèque d'images médicales, chaque image médicale représentant un signe, le procédé comprenant, postérieurement à l'étape d'identification de signes potentiels, une étape de recherche d'images de référence, dans laquelle, pour au moins l'un des signes potentiels, l'ensemble des images médicales représentant ledit au moins un signe potentiel sont recherchées dans la bibliothèque d'images médicales, lesdites images médicales formant les images de référence.

Chaque image médicale peut bien entendu représenter plusieurs signes. Une image peut représenter une situation considérée comme normale ou anormale. Dans un mode de réalisation particulier, l'étape de recherche d'images de référence comprend la recherche, pour au moins l'un des signes potentiels, d'une image représentant le signe potentiel dans une situation normale, et une image représentant le même signe potentiel dans une situation anormale.

Selon une forme particulière de réalisation, l'étape de recherche d'images de référence est réalisée postérieurement à l'étape d'identification de modalités techniques d'acquisition d'intérêt, et comprend, pour au moins l'un des signes potentiels, la recherche d'images médicales représentant ledit au moins un signe potentiel et étant acquises par l'une des modalités techniques d'acquisition d'intérêt. Les images de références sont ainsi présentées au praticien selon la même forme que celle qu'ils sont censés obtenir lors de l'acquisition des images.

L'ontologie médicale peut porter sur différents domaines médicaux et, notamment, sur les domaines gynécologiques et obstétriques. En particulier, l'ontologie médicale peut porter sur les grossesses ectopiques.

L'invention a également pour objet un dispositif apte à mettre en œuvre le procédé selon l'invention. Plus précisément, le dispositif peut comporter une mémoire informatique dans laquelle est enregistrée la base de connaissances, et des moyens de traitement aptes à réaliser les différentes étapes, notamment l'étape de recherche d'états pathologiques potentiels, l'étape d'identification de signes potentiels et, le cas échéant, l'étape d'identification de modalités techniques d'acquisition d'intérêt.

Selon une forme particulière de réalisation, le dispositif comporte en outre des moyens d'acquisition d'images médicales, apte à acquérir des images selon au moins l'une des modalités techniques d'acquisition d'images médicales de la classe imagerie.

L'invention a aussi pour objet un programme informatique comprenant des instructions exécutables par un ordinateur pour mettre en œuvre le procédé d'assistance à l'établissement d'un diagnostic d'un patient tel que décrit précédemment.

L'invention a enfin pour objet un support informatique comprenant des instructions exécutables par un ordinateur pour mettre en œuvre le procédé décrit précédemment.

### Description des figures

D'autres avantages et particularités de l'invention apparaîtront à la lecture de la description détaillée de mises en œuvre et de modes de réalisation nullement limitatifs, au regard de dessins annexés sur lesquels :
- la figure 1 représente schématiquement un exemple de base de connaissances informatique sur laquelle repose le procédé d'assistance à l'établissement d'un diagnostic selon l'invention ;
- la figure 2 représente un exemple d'ontologie médicale ;
- la figure 3 illustre un exemple de relations logiques entre un état pathologique et des signes ;
- la figure 4 illustre un exemple de relations logiques entre plusieurs états pathologiques ;
- la figure 5 représente un exemple de procédé d'assistance à l'établissement d'un diagnostic d'un patient selon l'invention.

### Description de modes de réalisation

Les modes de réalisation décrits ci-après étant nullement limitatifs, on pourra notamment considérer des variantes de l'invention ne comprenant qu'une sélection de caractéristiques décrites, par la suite isolées des autres caractéristiques décrites (même si cette sélection est isolée au sein d'une phrase comprenant ces autres caractéristiques), si cette sélection de caractéristiques est suffisante pour conférer un avantage technique ou pour différencier l'invention par rapport à l'état de la technique antérieure. Cette sélection comprend au moins une caractéristique, de préférence fonctionnelle sans détails structurels, ou avec seulement une partie des détails structurels si cette partie uniquement est suffisante pour conférer un avantage technique ou pour différencier l'invention par rapport à l'état de la technique antérieure.

Il est rappelé que le terme "signe" désigne tout phénomène lié à l'état d'un patient, et recouvre notamment les concepts de "signe fonctionnel", de "signe clinique", et de "signe paraclinique". Le terme "état pathologique" se rapporte à un état d'altération des fonctions, de la morphologie, ou de la santé d'un organe ou d'un organisme, se manifestant par la présence ou l'absence d'un ou plusieurs signes. Le terme "modalité technique d'acquisition d'une image médicale" désigne un ensemble de particularités techniques permettant d'obtenir une image médicale, apte à être comparée à une image de référence ou à des données de référence.

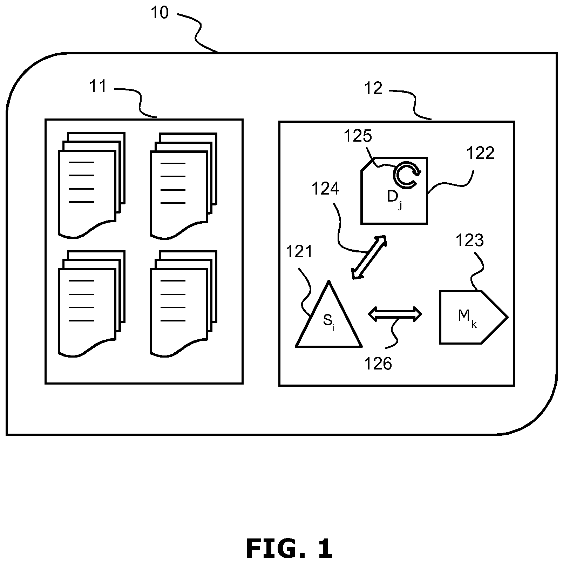

La figure 1 représente schématiquement une base de connaissances informatique sur laquelle repose le procédé d'assistance à l'établissement d'un diagnostic selon l'invention. La base de connaissances 10 est par exemple enregistrée dans une mémoire informatique d'un ordinateur ou d'un serveur informatique, ou sur un support de stockage amovible, tel qu'un disque compact (CD) ou une clé USB. Elle comprend une bibliothèque 11 d'images médicales et une ontologie médicale 12. Chaque image médicale représente un ou plusieurs signes, et est associée à un label indiquant différentes propriétés de l'image médicale. Le label indique par exemple la technique d'acquisition de l'image médicale (scanner, échographie, tomodensitométrie, etc.), l'organe du patient représenté, le positionnement de l'image relativement au patient, par exemple le plan de coupe dans lequel se situe l'image, pour une image plane, ou les frontières de l'image, pour une image tridimensionnelle. Le label peut aussi indiquer la présence de chaque signe observable sur l'image associée, et l'état clinique de ce signe. L'état clinique prend par exemple l'une des valeurs suivantes : "état normal", "état variant de la normale", "malformation", "inflammation", excroissance", etc. La bibliothèque 11 d'images médicales comporte par exemple des couples ou des ensembles d'images médicales, représentant un signe donné pour différents états cliniques. En particulier, l'une des images peut représenter le signe pour un état considéré comme normal.

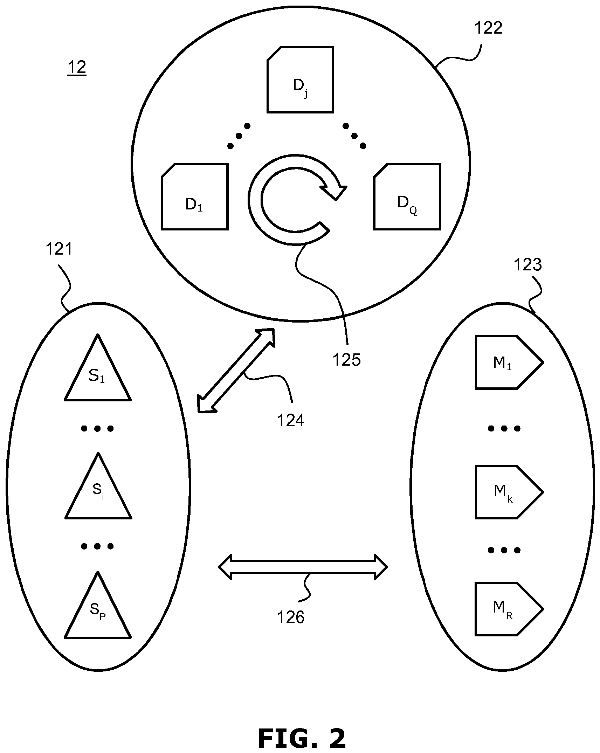

La figure 2 représente plus en détail l'ontologie médicale 12. L'ontologie médicale 12 comprend une liste 121 de signes, une liste 122 d'états pathologiques, et une liste 123 de modalités techniques d'acquisition d'images médicales. Par souci de facilité de lecture, les signes sont désignés individuellement ou globalement sous la référence Sᵢ, où l'indice i est un entier naturel compris entre un et P, les états pathologiques sont désignés individuellement ou globalement sous la référence Dⱼ, où l'indice j est un entier naturel compris entre un et Q, et les modalités techniques d'acquisition d'images médicales sont désignées individuellement ou globalement sous la référence Mₖ, où l'indice k est un entier naturel compris entre un et R. Les entiers P, Q et R sont des entiers naturels supérieurs ou égaux à deux. Ils peuvent être de l'ordre de quelques dizaines, de quelques centaines, voire de plusieurs milliers. L'ensemble des signes Sᵢ forme une classe "signe", l'ensemble des états pathologiques Dⱼ forme une classe "état pathologique", et l'ensemble des modalités techniques d'acquisition d'images médicales forme une classe "imagerie".

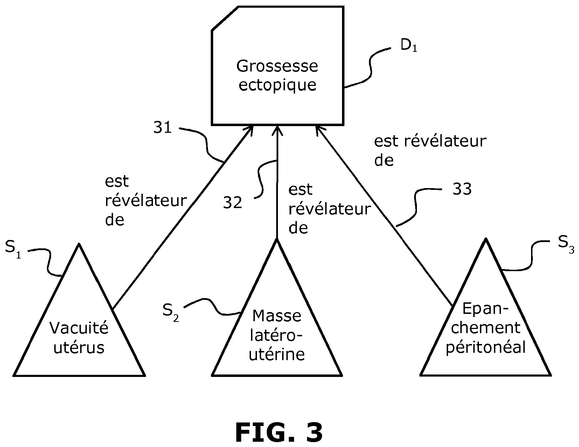

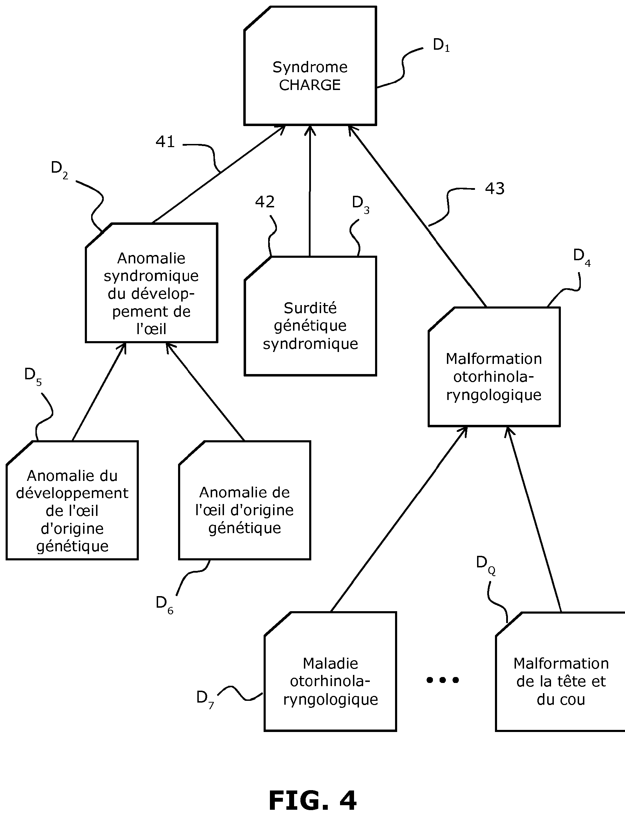

L'ontologie médicale 12 comprend de plus un premier ensemble 124 de relations logiques entre les signes Sᵢ et les états pathologiques Dⱼ, un deuxième ensemble 125 de relations logiques entre les états pathologiques Dⱼ, et un troisième ensemble 126 de relations logiques entre les signes Sᵢ et les modalités techniques d'acquisition d'images médicales Mₖ. Chaque relation logique du premier ensemble 124 établit un lien de corrélation entre un signe Sᵢ et un état pathologique Dⱼ, comme expliqué plus en détail en référence à la figure 3. Chaque relation logique du deuxième ensemble 125 définit un lien de parenté entre un état pathologique donné Dⱼ, et un état pathologique parent Dₗ de cet état pathologique donné, comme expliqué plus en détail à la figure 4. Chaque relation logique du troisième ensemble 126 définit une modalité technique d'acquisition d'une image médicale Mₖ permettant d'observer l'un des signes Sᵢ. Un signe Sᵢ peut être observable par plusieurs modalités techniques d'acquisition d'une image médicale Mₖ. Par ailleurs, une modalité technique d'acquisition d'une image médicale Mₖ peut permettre d'observer plusieurs signes Sᵢ.

La figure 3 illustre un exemple de relations logiques entre un état pathologique D₁ et des signes S₁, S₂, et S₃, dans le domaine gynécologique. À titre d'exemple, on considère que l'état pathologique D₁ est une grossesse ectopique et que les signes S₁, S₂ et S₃ sont respectivement une vacuité de l'utérus, une masse latéro-utérine, et un épanchement péritonéal. Chaque signe Sᵢ est révélateur de l'état pathologique D₁. Les relations logiques 31, 32, 33 sont donc définies en conséquence. Il est possible d'attribuer à chaque relation logique un coefficient représentatif de la probabilité qu'un signe soit associé à un état pathologique. Lors de l'exécution du procédé selon l'invention, des états pathologiques potentiels peuvent ainsi être mis en avant par rapport à d'autres.

La figure 4 illustre un exemple de relations logiques entre plusieurs états pathologiques, dans le domaine de la médecine fœtale. À titre d'exemple, on considère que l'état pathologique D₁ est le syndrome CHARGE, et qu'il s'agit d'un état pathologique d'ordre un. Cet état pathologique D₁ est l'état pathologique fils de trois états pathologiques D₂, D₃ et D₄, correspondant respectivement à une anomalie syndromique du développement de l'œil, une surdité génétique syndromique, et une malformation otorhinolaryngologique. Ces états pathologiques D₂-D₄ sont appelés états pathologiques parents, ou états pathologiques premiers parents, dans la mesure où ils sont directement liés à l'état pathologique D₁. Ils forment des états pathologiques d'ordre deux. L'état pathologique D₂, (anomalie syndromique du développement de l'œil), est le fils de deux états pathologiques parents D₅ et D₆, correspondant respectivement à une anomalie du développement de l'œil d'origine génétique et une anomalie de l'œil d'origine génétique. Ces états pathologiques constituent des états pathologiques d'ordre trois vis-à-vis de l'état pathologique D₁. De même, l'état pathologique D₄, (malformation otorhinolaryngologique), est l'état pathologique fils des états pathologiques D₇ (maladie otorhinolaryngologique) à D_{Q} (malformation de la tête et du cou). De manière analogue aux relations entre les signes et les états pathologiques, les relations logiques entre les états pathologiques peuvent comporter des coefficients représentatifs d'une probabilité de parenté.

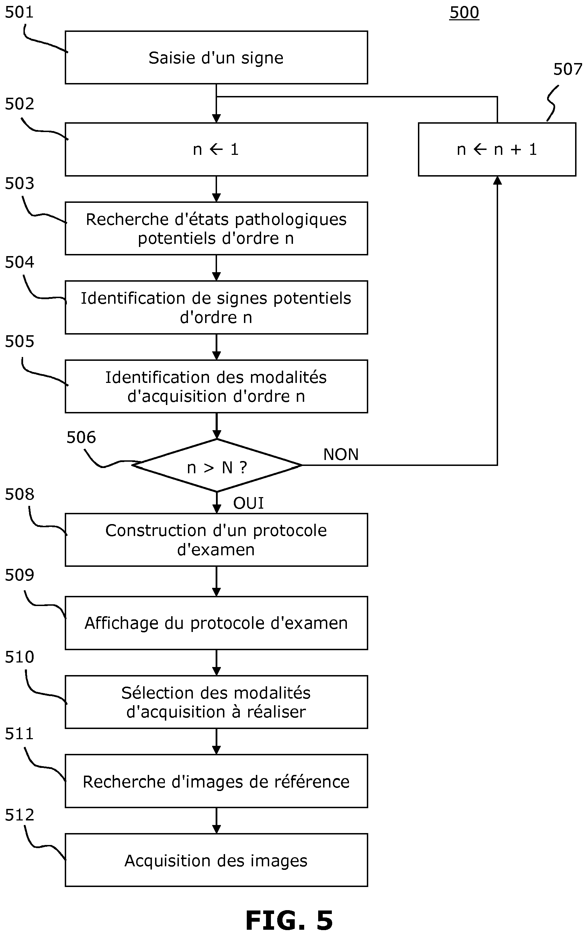

La figure 5 représente un exemple de procédé d'assistance à l'établissement d'un diagnostic d'un patient selon l'invention. Le procédé 500 comprend une première étape 501, dans laquelle un ou plusieurs signes Sᵢ sont saisis par un opérateur dans un dispositif mettant en œuvre le procédé, tel qu'un ordinateur. Ces signes peuvent typiquement avoir été identifiés ou supposés par un opérateur. Ils sont ainsi appelés "signes identifiés".

Dans des étapes ultérieures, des états pathologiques potentiels d'ordre un à N sont recherchés. À cet effet, dans une deuxième étape 502, une variable n prend la valeur un.

Dans une étape ultérieure 503, l'ensemble des états pathologiques potentiels Dⱼ d'ordre n sont recherchés dans la classe "état pathologique". Lors de la première itération de cette étape 503, où n a la valeur un, la recherche est effectuée au moyen du premier ensemble 124 de relations logiques. Lors des itérations suivantes de l'étape 503, où n prend une valeur entière comprise entre deux et N, la recherche est effectuée au moyen du deuxième ensemble 125 de relations logiques. L'étape 503 de recherche des états pathologiques potentiels Dⱼ aboutit à une liste d'états pathologiques potentiels de différents ordres. L'information relative à l'ordre de chaque état pathologique peut être conservée, dans un objectif de hiérarchisation, comme expliqué plus loin.

Dans une quatrième étape 504, l'ensemble des signes potentiels Sᵢ d'ordre n révélateurs d'un état pathologique potentiel Dⱼ d'ordre n sont identifiés dans la classe signes. Cette étape 504 est réalisée au moyen du premier ensemble 124 de relations logiques. L'étape 504 peut comporter un classement des signes potentiels Sᵢ identifiés, en fonction de l'ordre de l'état pathologique potentiel Dⱼ qui les a révélés. Avantageusement, lorsqu'un signe potentiel Sᵢ est identifié pour différents ordres, il est possible de ne conserver que le signe potentiel d'ordre le moins élevé.

Dans une étape 505, l'ensemble des modalités techniques d'acquisition des images médicales Mₖ permettant d'observer les signes potentiels Sᵢ d'ordre n sont identifiés. Plus précisément, pour chaque signe potentiel Sᵢ d'ordre n, toutes les modalités techniques d'acquisition des images médicales Mₖ sont identifiées dans la classe signe, au moyen du troisième ensemble 126 de relations logiques. Ces modalités d'acquisition sont appelées modalités techniques d'acquisition d'intérêt. L'étape 505 peut comporter un classement des modalités d'acquisition d'intérêt Mₖ en fonction de l'ordre associé au signe potentiel Sᵢ considéré, de manière à permettre de classer ces modalités d'acquisition Mₖ selon qu'elles sont plus ou moins susceptibles de montrer des signes potentiels Sᵢ proches du signe initial, identifié par l'opérateur.

Dans une étape 506, il est vérifié si la recherche d'états pathologiques potentiels, et de leurs signes potentiels associés, a exploré suffisamment de voies possibles. En pratique, il est vérifié si la variable n est inférieure ou égale à N, ou strictement supérieure à N. Dans le premier cas, cette variable est incrémentée d'une unité dans une étape 507, et les étapes 502 à 506 sont réitérées avec cette nouvelle valeur de n. Dans le deuxième cas, il est mis fin à la recherche de nouveaux états pathologiques potentiels, de nouveaux signes potentiels associés, et de nouvelles modalités d'acquisition d'intérêt associées.

Le procédé exécute alors une étape 508, dans laquelle un protocole d'examen est construit. Ce protocole d'examen consiste à lister l'ensemble des modalités techniques d'acquisition d'intérêt Mₖ, de préférence en fonction de leur classement. Le protocole d'examen ainsi construit peut être affiché à l'opérateur dans une étape 509.

Dans une étape 510, les modalités techniques d'acquisition d'images à mettre en œuvre sont sélectionnées parmi les modalités techniques d'intérêt. La sélection peut être réalisée par un opérateur, ou de façon automatique par un algorithme. Elle consiste par exemple à sélectionner les modalités d'acquisition présentant l'ordre le plus faible. En outre, les modalités d'acquisition permettant d'observer le plus de signes potentiels sur une même image, ou impliquant l'utilisation d'un seul appareil d'imagerie médicale, peuvent être sélectionnées au détriment de modalités d'acquisition aboutissant à l'observation d'un unique signe potentiel, ou de modalités d'acquisitions impliquant l'utilisation de différents appareils d'imagerie médicale.

Dans une étape optionnelle 511, une ou plusieurs images de référence peuvent être recherchées en fonction des modalités d'acquisition à mettre en œuvre. L'étape 511 consiste à rechercher, dans la bibliothèque 11 d'images médicales, celles correspondant aux modalités techniques d'acquisition à mettre en œuvre, et qui représentent donc des signes potentiels. Cette recherche peut être effectuée via le label associé à chaque image. L'opérateur devant effectuer l'acquisition des images médicales dispose ainsi d'images de référence pour lui permettre de vérifier que l'image acquise correspond bien à celle attendue pour l'établissement ultérieur d'un diagnostic.

Dans une étape 512, des images médicales sont acquises. Ces images médicales sont acquises conformément aux modalités techniques d'acquisition à mettre en œuvre. L'acquisition des images peut être réalisée par l'opérateur, ou de manière automatique, par un ou plusieurs dispositifs d'acquisition d'images.

Bien sûr, l'invention n'est pas limitée aux exemples qui viennent d'être décrits et de nombreux aménagements peuvent être apportés à ces exemples sans sortir du cadre de l'invention, tel que défini par les revendications jointes. En particulier, les signes de l'ontologie ne sont pas nécessairement observables par une image, mais peuvent consister en tout type de phénomène lié à l'état d'un patient, tel que la température corporelle du patient, ou une propriété physique ou biochimique d'un fluide corporelle du patient. Ainsi, l'étape d'identification de modalités techniques d'acquisition d'images médicales peut consister, plus généralement, en une étape d'identification de modalités techniques d'acquisition de données médicales.

## Revendications

1. Procédé d'assistance à l'établissement d'un diagnostic d'un patient mis en œuvre par un dispositif, à partir d'au moins un signe identifié et saisi par un opérateur dans ce dispositif (501) et en s'appuyant sur une base de connaissances informatique (10) comportant une ontologie médicale (12), l'ontologie médicale comprenant :
▪ une liste (121) de signes (Sᵢ), formant une classe "signe",
▪ une liste (122) d'états pathologiques (Dⱼ), formant une classe "état pathologique", et
▪ un premier ensemble (124) de relations logiques entre les signes et les états pathologiques, chaque relation logique établissant un lien de corrélation entre un signe et un état pathologique,
le procédé (500) comprenant :
▪ une étape (503) de recherche d'états pathologiques potentiels, dans laquelle l'ensemble des états pathologiques liés par un lien de corrélation avec au moins l'un des signes identifiés sont recherchés dans la classe état pathologique au moyen du premier ensemble (124) de relations logiques, lesdits états pathologiques formant les états pathologiques potentiels, et
▪ une étape (504) d'identification de signes potentiels, dans laquelle, pour chaque état pathologique potentiel, l'ensemble des signes liés par un lien de corrélation avec ledit état pathologique potentiel sont identifiés dans la classe signe au moyen du premier ensemble (124) de relations logiques, lesdits signes formant les signes potentiels,
**caractérisé en ce que** l'ontologie médicale (12) comprend en outre :
▪ une liste (123) de modalités techniques d'acquisition d'images médicales (Mₖ), formant une classe "imagerie", chaque modalité technique d'acquisition d'une image médicale définissant un type de technologie d'acquisition d'images médicales, et un positionnement de l'image médicale sur le patient, et
▪ un troisième ensemble (126) de relations logiques entre les signes et les modalités techniques d'acquisition d'images médicales, chaque relation logique définissant une modalité technique d'acquisition d'une image médicale permettant d'observer un signe,
le procédé comprenant en outre :
▪ une étape (505) d'identification de modalités techniques d'acquisition d'intérêt, dans laquelle, pour chaque signe potentiel, l'ensemble des modalités techniques d'acquisition d'images médicales permettant d'observer ledit signe potentiel sont identifiées dans la classe imagerie au moyen du troisième ensemble (126) de relations logiques, lesdites modalités techniques d'acquisition d'images médicales formant les modalités techniques d'acquisition d'intérêt.

2. Procédé selon la revendication 1, dans lequel le premier ensemble (124) de relations logiques comprend des relations logiques selon chacune desquelles un signe (Sᵢ) est révélateur d'un état pathologique (Dⱼ).

3. Procédé selon la revendication 2, dans lequel l'ontologie médicale (12) comprend, en outre, un deuxième ensemble (125) de relations logiques entre des états pathologiques de la classe état pathologique, chaque relation logique définissant un lien de parenté entre un état pathologique donné et un état pathologique parent de cet état pathologique donné, l'étape (503) de recherche d'états pathologiques potentiels comprenant :
▪ une recherche d'états pathologiques potentiels d'ordre un, dans laquelle l'ensemble des états pathologiques révélés par au moins l'un des signes identifiés sont recherchés, lesdits états pathologiques formant les états pathologiques potentiels d'ordre un, et
▪ une recherche d'états pathologiques potentiels d'ordre deux à N, où N est un entier naturel supérieur ou égal à deux, la recherche d'états pathologiques potentiels d'ordre n, où n est un entier naturel compris entre deux et N, comprenant, pour chaque état pathologique potentiel d'ordre n-1, une recherche de l'ensemble des états pathologiques parents de cet état pathologique potentiel d'ordre n-1 au moyen du deuxième ensemble (125) de relations logiques, lesdits états pathologiques parents formant les états pathologiques potentiels d'ordre n, l'étape (504) d'identification de signes potentiels comprenant une identification de l'ensemble des signes révélateurs d'un état pathologique potentiel d'ordre un, et/ou d'un état pathologique potentiel d'ordre n.

4. Procédé selon la revendication 3, dans lequel l'étape (504) d'identification de signes potentiels comprend un classement de ces signes potentiels, selon lequel l'ensemble des signes potentiels révélateurs d'un état pathologique potentiel d'ordre m, où m est un entier naturel compris entre un et N, forment les signes potentiels d'ordre m.

5. Procédé selon la revendication 4, dans lequel l'étape (505) d'identification de modalités techniques d'acquisition d'intérêt comprend un classement de ces modalités techniques d'acquisition d'intérêt, selon lequel l'ensemble des modalités techniques d'acquisition d'images médicales d'intérêt permettant d'observer un signe potentiel d'ordre m forment les modalités techniques d'acquisition d'intérêt d'ordre m.

6. Procédé selon la revendication 5 comprenant, en outre, une étape (508) de construction d'un protocole d'examen, dans lequel les modalités techniques d'acquisition d'intérêt sont hiérarchisées selon leur ordre, les modalités techniques d'acquisition d'intérêt d'ordre m étant prioritaires par rapport aux modalités techniques d'acquisition d'intérêt d'ordre m+1.

7. Procédé selon la revendication 6, dans lequel les modalités techniques d'acquisition d'intérêt de même ordre sont hiérarchisées selon leur occurrence parmi l'ensemble des modalités techniques d'acquisition d'intérêt de cet ordre, ou parmi l'ensemble des modalités techniques d'acquisition d'intérêt des différents ordres.

8. Procédé selon l'une des revendications 4 à 7 comprenant, en outre, une étape d'affichage des modalités techniques d'acquisition d'intérêt.

9. Procédé selon l'une des revendications 4 à 8, comprenant, en outre :
▪ une étape (510) de sélection de modalités techniques d'acquisition à mettre en œuvre, dans laquelle au moins l'une des modalités techniques d'acquisition d'intérêt est sélectionnée, l'ensemble des modalités techniques d'acquisition d'intérêt sélectionnées formant les modalités techniques d'acquisition à mettre en œuvre, et
▪ une étape (512) d'acquisition d'une ou plusieurs images médicales, chaque image médicale étant acquise selon l'une des modalités techniques d'acquisition à mettre en œuvre.

10. Procédé selon l'une des revendications précédentes, dans lequel la base de connaissances informatique (10) comporte, en outre, une bibliothèque (11) d'images médicales, chaque image médicale représentant un signe, le procédé comprenant, postérieurement à l'étape (504) d'identification de signes potentiels, une étape (511) de recherche d'images de référence, dans laquelle, pour au moins l'un des signes potentiels, l'ensemble des images médicales représentant ledit au moins un signe potentiel sont recherchées dans la bibliothèque (11) d'images médicales, lesdites images médicales formant les images de référence.

11. Procédé selon la revendication 10, prise avec l'une des revendications 1 à 9, dans lequel l'étape (511) de recherche d'images de référence est réalisée postérieurement à l'étape (505) d'identification de modalités techniques d'acquisition d'intérêt, et comprend, pour au moins l'un des signes potentiels, la recherche d'images médicales représentant ledit au moins un signe potentiel et étant acquises par l'une des modalités techniques d'acquisition d'intérêt.

12. Procédé selon l'une des revendications précédentes, dans lequel l'ontologie médicale (12) porte sur les grossesses ectopiques.

13. Programme informatique comprenant des instructions exécutables par un ordinateur pour mettre en œuvre le procédé selon l'une des revendications précédentes.

14. Support informatique comprenant des instructions exécutables par un ordinateur pour mettre en œuvre le procédé selon l'une des revendications 1 à 12.

## Patentansprüche

1. Verfahren zur Unterstützung bei der Erstellung einer Diagnose eines Patienten, das durch eine Vorrichtung, ausgehend von mindestens einem Zeichen, das von einem Bediener in dieser Vorrichtung (501) identifiziert und eingegeben wird, implementiert wird und sich auf eine EDV-Wissensbasis (10) stützt, die eine medizinische Ontologie (12) enthält, wobei die medizinische Ontologie umfasst:
▪ eine Liste (121) von Zeichen (Sᵢ), die eine "Zeichen"-Klasse bilden,
▪ eine Liste (122) von pathologischen Zuständen (Dⱼ), die eine Klasse "pathologischer Zustand" bilden, und
▪ einen ersten Satz (124) von logischen Beziehungen zwischen Zeichen und pathologischen Zuständen, wobei jede logische Beziehung eine Korrelationsverbindung zwischen einem Zeichen und einem pathologischen Zustand herstellt, wobei das Verfahren (500) umfasst:
▪ einen Schritt (503) der Suche nach potentiellen pathologischen Zuständen, bei dem der Satz der pathologischen Zustände, die durch eine Korrelationsverbindung mit mindestens einem der identifizierten Zeichen verbunden sind, in der Klasse der pathologischen Zustände mittels des ersten Satzes (124) logischer Beziehungen gesucht wird, wobei die pathologischen Zustände die potentiellen pathologischen Zustände bilden, und
▪ einen Schritt (504) des Identifizierens potentieller Zeichen, wobei für jeden potentiellen pathologischen Zustand der Satz von Zeichen, die mit dem potentiellen pathologischen Zustand korreliert sind, mit Hilfe des ersten Satzes (124) logischer Beziehungen in der Zeichenklasse identifiziert wird, wobei die Zeichen die potentiellen Zeichen bilden,
**dadurch gekennzeichnet, dass** die medizinische Ontologie (12) ferner umfasst:
▪ eine Liste (123) von technischen Modalitäten zur Erfassung medizinischer Bilder (Mₖ), die eine Bildgebungsklasse bilden, wobei jede technische Modalität zur Erfassung eines medizinischen Bildes eine Art von medizinischer Bilderfassungstechnologie und eine Positionierung des medizinischen Bildes am Patienten definiert, und
▪ einen dritten Satz (126) von logischen Beziehungen zwischen den Zeichen und den technischen Modalitäten zur Erfassung medizinischer Bilder, wobei jede logische Beziehung eine technische Modalität der Erfassung medizinischer Bilder definiert, die das Beobachten eines Zeichens zulässt, wobei das Verfahren ferner umfasst:
▪ einen Schritt (505) zum Identifizieren technischer Modalitäten der Erfassung von Interessensbereichen, wobei für jedes potentielle Zeichen der Satz der technischen Modalitäten der Erfassung medizinischer Bilder, die die Beobachtung des potentiellen Zeichens ermöglichen, in der Bildgebungsklasse mittels des dritten Satzes (126) logischer Beziehungen identifiziert wird, wobei die technischen Modalitäten der Erfassung medizinischer Bilder die technischen Modalitäten der Erfassung von Interessensbereichen bilden.

2. Verfahren nach Anspruch 1, bei dem der erste Satz (124) von logischen Beziehungen logische Beziehungen umfasst, nach denen jeweils ein Zeichen (Sᵢ) einen pathologischen Zustand (Dⱼ) anzeigt.

3. Verfahren nach Anspruch 2, wobei die medizinische Ontologie (12) ferner einen zweiten Satz (125) von logischen Beziehungen zwischen pathologischen Zuständen der Klasse der pathologischen Zustände umfasst, wobei jede logische Beziehung eine verwandtschaftliche Beziehung zwischen einem gegebenen pathologischen Zustand und einem verwandten pathologischen Zustand dieses gegebenen pathologischen Zustandes definiert, wobei der Schritt (503) des Suchens nach potenziellen pathologischen Zuständen umfasst:
▪ eine Suche nach potentiellen pathologischen Zuständen der Ordnung eins, bei der der Satz der pathologischen Zustände gesucht wird, die durch mindestens eines der identifizierten Zeichen aufgedeckt werden, wobei die pathologischen Zustände die potentiellen pathologischen Zustände der Ordnung eins bilden, und
▪ eine Suche nach potentiellen pathologischen Zuständen der Ordnung zwei bis N, wobei N eine natürliche Zahl größer oder gleich zwei ist, wobei die Suche nach potentiellen pathologischen Zuständen der Ordnung n, wobei n eine natürliche Zahl zwischen zwei und N ist, für jeden potentiellen pathologischen Zustand der Ordnung n-1 eine Suche nach dem Satz von verwandten pathologischen Zuständen dieses potentiellen pathologischen Zustands der Ordnung n-1 mittels des zweiten Satzes (125) logischer Beziehungen umfasst, wobei die verwandten pathologischen Zustände die potentiellen pathologischen Zustände der Ordnung n bilden, wobei der Schritt (504) des Identifizierens potentieller Zeichen das Identifizieren des Satzes an Zeichen umfasst, die einen potentiellen pathologischen Zustand der Ordnung eins und/oder einen potentiellen pathologischen Zustand der Ordnung n aufdecken.

4. Verfahren nach Anspruch 3, wobei der Schritt (504) zum Identifizieren potentieller Zeichen das Klassifizieren solcher potentiellen Zeichen umfasst, gemäß dem der Satz potentieller Zeichen, die einen potentiellen pathologischen Zustand der Ordnung m aufdecken, wobei m eine natürliche Zahl zwischen eins und N ist, die potentiellen Zeichen der Ordnung m bildet.

5. Verfahren nach Anspruch 4, bei dem der Schritt (505) zum Identifizieren technischer Modalitäten der Erfassung von Interessensbereichen eine Klassifizierung dieser technischen Modalitäten der Erfassung von Interessensbereichen umfasst, nach der der Satz von technischen Modalitäten der Erfassung von medizinischen Bildern von Interesse, der es ermöglicht ein potentielles Zeichen der Ordnung m zu beobachten, die technischen Methoden der Erfassung von Interessensbereichen der Ordnung m bildet.

6. Verfahren nach Anspruch 5, ferner umfassend einen Schritt (508) zum Erstellen eines Prüfprotokolls, bei dem die technischen Modalitäten der Erfassung von Interessensbereichen gemäß ihrer Ordnung priorisiert sind, wobei die technischen Modalitäten der Erfassung von Interessensbereichen der Ordnung m Vorrang vor den technischen Modalitäten der Erfassung von Interessensbereichen der Ordnung m+1 haben.

7. Verfahren nach Anspruch 6, bei dem die technischen Modalitäten der Erfassung von Interessensbereichen derselben Ordnung nach ihrem Auftreten unter den technischen Modalitäten der Erfassung von Interessensbereichen dieser Ordnung oder unter dem Satz von technischen Modalitäten der Erfassung von Interessensbereichen verschiedener Ordnungen priorisiert sind.

8. Verfahren nach einem der Ansprüche 4 bis 7, ferner umfassend einen Schritt des Anzeigens der technischen Modalitäten der Erfassung von Interessensbereichen.

9. Verfahren nach einem der Ansprüche 4 bis 8, ferner umfassend:
▪ einen Schritt (510) der Auswahl der zu implementierenden technischen Modalitäten der Erfassung, in dem mindestens eine der technischen Modalitäten der Erfassung von Interessensbereichen ausgewählt wird, wobei der Satz der ausgewählten technischen Modalitäten der Erfassung von Interessensbereichen die zu implementierenden technischen Modalitäten der Erfassung von Interessensbereichen bildet und
▪ einen Schritt (512) der Erfassung eines oder mehrerer medizinischer Bilder, wobei jedes medizinische Bild gemäß einer zu implementierenden technischen Modalität der Erfassung erfasst wird.

10. Verfahren nach einem der vorhergehenden Ansprüche, bei dem die EDV-Wissensbasis (10) außerdem eine Bibliothek (11) medizinischer Bilder enthält, wobei jedes medizinische Bild ein Zeichen darstellt, wobei das Verfahren nach dem Schritt (504) der Identifizierung potentieller Zeichen einen Schritt (511) der Suche nach Referenzbildern umfasst, bei dem für mindestens eines der potentiellen Zeichen der Satz der medizinischen Bilder, die das mindestens eine potentielle Zeichen darstellen, in der Bibliothek (11) medizinischer Bilder gesucht wird, wobei die medizinischen Bilder die Referenzbilder bilden.

11. Verfahren nach Anspruch 10, zusammen mit einem der Ansprüche 1 bis 9, bei dem der Schritt (511) der Suche nach Referenzbildern im Anschluss an den Schritt (505) der Identifizierung der technischen Modalitäten der Erfassung von Interessensbereichen durchgeführt wird und für mindestens eines der potenziellen Zeichen die Suche nach medizinischen Bildern umfasst, die das mindestens eine potenzielle Zeichen darstellen und durch eine der technischen Modalitäten der Erfassung von Interessensbereichen erfasst werden.

12. Verfahren nach einem der vorhergehenden Ansprüche, bei dem sich die medizinische Ontologie (12) auf Eileiterschwangerschaften bezieht.

13. Computerprogramm, das Anweisungen umfasst, die von einem Computer zum Implementieren des Verfahrens gemäß einem der vorhergehenden Ansprüche ausführbar sind.

14. Computermedium, das Anweisungen umfasst, die von einem Computer zum Implementieren des Verfahrens gemäß einem der Ansprüche 1 bis 12 ausführbar sind.

## Claims

1. Method for assistance with the establishment of a diagnosis of a patient implemented by a device, starting from at least one identified sign and entered by an operator into said device (501) and based on a computerized knowledge database (10) comprising a medical ontology (12), the medical ontology comprising:
▪ a list (121) of signs (Sᵢ), forming a "sign" class,
▪ a list (122) of pathological states (Dⱼ), forming a "pathological state" class, and
▪ a first set (124) of logical relationships between the signs and the pathological states, each logical relationship establishing a correlative link between a sign and a pathological state,
the method (500) comprising:
▪ a step (503) of searching for potential pathological states, in which all of the pathological states linked by a correlative link to at least one of the identified signs are sought in the pathological state class by means of the first set (124) of logical relationships, said pathological states forming the potential pathological states, and
▪ a step (504) of identifying potential signs in which, for each potential pathological state, all of the signs linked by a correlative link to said potential pathological state are identified in the sign class by means of the first set (124) of logical relationships, said signs forming the potential signs,
**characterized in that** the medical ontology (12) further comprises:
▪ a list (123) of technical methods for acquisition of medical images (Mₖ), forming an "imaging" class, each technical method for acquisition of a medical image defining a type of technology for acquisition of medical images, and a positioning of the medical image on the patient, and
▪ a third set (126) of logical relationships between the signs and the technical methods for acquisition of medical images, each logical relationship defining a technical method for acquisition of a medical image making it possible to observe a sign,
the method further comprising:
▪ a step (505) of identifying technical methods for acquisition of interest, in which for each potential sign, all of the technical methods for acquisition of medical images making it possible to observe said potential sign are identified in the imaging class by means of the third set (126) of logical relationships, said technical methods for acquisition of medical images forming the technical methods for acquisition of interest..

2. Method according to claim 1, in which the first set (124) of logical relationships comprises logical relationships according to each of which a sign (Sᵢ) is indicative of a pathological state (Dⱼ).

3. Method according to claim 2, in which the medical ontology (12) further comprises a second set (125) of logical relationships between pathological states of the pathological state class, each logical relationship defining a kinship link between a given pathological state and a parent pathological state of this given pathological state, the step (503) of searching for potential pathological states comprising:
▪ searching for potential pathological states of order one, in which all of the pathological states indicated by at least one of the identified signs are sought, said pathological states forming the potential pathological states of order one, and
▪ searching for potential pathological states of order two to N, where N is a natural integer greater than or equal to two, the search for potential pathological states of order n, where n is a natural integer comprised between two and N, comprising, for each potential pathological state of order n-1, searching for all of the parent pathological states of this potential pathological state of order n-1 by means of the second set (125) of logical relationships, said parent pathological states forming the potential pathological states of order n,
the step (504) of identifying potential signs comprising an identification of all of the signs indicative of a potential pathological state of order one and/or a potential pathological state of order n.

4. Method according to claim 3, in which the step (504) of identifying potential signs comprises a classification of these potential signs, according to which all of the potential signs indicative of a potential pathological state of order m, where m is a natural integer comprised between one and N, form the potential signs of order m.

5. Method according to claim 4, in which the step (505) of identifying technical methods for acquisition of interest comprises a classification of these technical methods for acquisition of interest, according to which all of the technical methods for acquisition of medical images making it possible to observe a potential sign of order m form the technical methods for acquisition of interest of order m.

6. Method according to claim 5, further comprising a step (508) of constructing an examination protocol, in which the technical methods for acquisition of interest are hierarchized according to their order, the technical methods for acquisition of interest of order m having priority over the technical methods for acquisition of interest of order m+1.

7. Method according to claims 6, in which the technical methods for acquisition of interest of the same order are hierarchized according to their occurrence among all of the technical methods for acquisition of interest of this order, or among all of the technical methods for acquisition of interest of the different orders.

8. Method according to one of claims 4 to 7, further comprising a step of displaying the technical methods for acquisition of interest.

9. Method according to one of claims 4 to 8, further comprising:
▪ a step (510) of selecting technical methods for acquisition to be implemented, in which at least one of the technical methods for acquisition of interest is selected, all of the technical methods for acquisition of interest selected forming the technical methods for acquisition to be implemented, and
▪ a step (512) of acquisition of one or more medical images, each medical image being acquired according to one of the technical methods for acquisition to be implemented.

10. Method according to one of the preceding claims, in which the computerized knowledge database (10) further comprises a library (11) of medical images, each medical image representing a sign, the method comprising, after the step (504) of identifying potential signs, a step (511) of searching for reference images in which, in the case of at least one of the potential signs, all of the medical images representing said at least one potential sign are sought in the library (11) of medical images, said medical images forming the reference images.

11. Method according to claim 10, together with one of claims 1 to 9, in which the step (511) of searching for reference images is carried out after the step (505) of identifying technical methods for acquisition of interest, and comprises, in the case of at least one of the potential signs, searching for medical images representing said at least one potential sign and being acquired by means of one of the technical methods for acquisition of interest.

12. Method according to one of the preceding claims, in which the medical ontology (12) relates to ectopic pregnancies.

13. Computer program comprising computer-executable instructions for implementing the method according to one of the preceding claims.

14. Computer medium comprising computer-executable instructions for implementing the method according to one of claims 1 to 12.
